(19) Europäisches Patentamt
European Patent Office
Office européen des brevets

(11) Publication number: **0 288 055 B1**

(12) **EUROPEAN PATENT SPECIFICATION**

(45) Date of publication of patent specification: **15.07.92**

(51) Int. Cl.$^5$: **A61K 37/66**, //(A61K37/66, 31:415,31:195,31:13)

(21) Application number: **88106401.8**

(22) Date of filing: **21.04.88**

# (54) Use of ODC inhibitors, dacarbazine, and interferon, in the treatment of malignant melanoma.

(30) Priority: **23.04.87 US 41891**

(43) Date of publication of application:
**26.10.88 Bulletin 88/43**

(45) Publication of the grant of the patent:
**15.07.92 Bulletin 92/29**

(84) Designated Contracting States:
**AT BE CH DE ES FR GB GR IT LI LU NL SE**

(56) References cited:
**US-A- 4 499 072**

**J. of Interferon Research 5 (1985) p. 541-550**

(73) Proprietor: **MERRELL DOW PHARMACEUTICALS INC.**
**2110 East Galbraith Road**
**Cincinnati Ohio 45215-6300(US)**

(72) Inventor: **Sunkara, Sai P.**
**9629 Linfield Drive**
**Cincinnati Ohio 45242(US)**

(74) Representative: **Sgarbi, Renato et al**
**GRUPPO LEPETIT S.p.A. Patent and Trademark Department Via Roberto Lepetit, 34**
**I-21040 Gerenzano (Varese)(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to a pharmaceutical product containing an ornithine decarboxylase inhibitor, Interferon and Dacarbazine as a combined preparation for simultaneous or sequential use in treating rapidly-proliferating cell-growth disease states.

That rapidly-proliferating cell-growth disease states are widespread throughout the world and affect a significant proportion of the population and that such diseases have been the subject of intensive research efforts is well known. Unfortunately, despite such efforts and despite some successes, the overall control of these diseases has not been too satisfactory. Recently, however, the pursuit in the alleviation and treatment of such disease states has shown promise by the discovery of irreversible inhibitors of enzymes which relate to the biosynthesis of the polyamines necessary for cell growth. Particularly useful enzyme inhibitors are those which produce in vivo irreversible inhibition of ornithine decarboxylase (ODC), the enzyme which catalyzes the decarboxylation of ornithine to putrescine.

The decarboxylation of ornithine to putrescine is the first step in the biosynthesis of the polyamines known as spermidine and spermine. Spermidine is formed by the transfer of an activated aminopropyl moiety from decarboxylated S-adenosyl-S-methionine to putrescine, while spermine is formed by the transfer of a second amino-propyl group to spermidine. Decarboxylated S-adenosyl-methionine-cysteamine is formed by the decarboxylation of S-adenosylmethionine (SAM), a reaction catalyzed by the enzyme S-adenosylmethionine decarboxylase (SAM-DC). Since putrescine is a precursor of the polyamines, it is seen that blockade of the conversion of ornithine to putrescine, such as by inhibition of ODC, can provide a method for regulating the cellular levels of the polyamines.

The polyamines, which are found in animal tissues and microorganisms, are known to play an important role in cell growth and proliferation. The induction of cell growth and proliferation is associated with a marked increase in ODC activity and an increase in the levels of putrescine and the polyamines. Although the exact the role of the polyamines in cell growth and proliferation is not known, it appears that the polyamines may facilitate macro-molecular processes such as DNA, RNA, or protein synthesis. Polyamine levels are known to be high in the testes, ventral prostate, and thymus as well as in psoriatic skin lesions and in other cells undergoing rapid growth processes.

It is also well known that the rapid proliferation of tumor tissue is marked by an abnormal elevation of polyamine levels. Hence, the polyamines may play an important role in the maintenance of tumor growth. It is thus believed that the ODC inhibitors may exert their therapeutic effect by blocking the formation of the polyamines and thereby slowing, interrupting, or arresting the proliferation and metastases of the tumor tissue.

In addition to the rather recent discovery concerning the use of irreversible inhibitors of ornithine decarboxylase, particularly with such compounds as α-di-fluoromethyl ornithine, the methyl and ethyl esters of monofluoromethyl dehydroornithine, the R,R-isomer of methyl acetylenic putrescine, combinations of these ODC inhibitors with chemical cytotoxic agents has also shown promise in the treatment of diseases characterized by the rapid proliferation of tumor tissue. Indeed, in the treatment of a malignant neoplastic disease it has been reported that α-halomethyl ornithines capable of irreversible ODC inhibition, in combination with known cytotoxic agents have demonstrated some synergistic effects in the treatment of such disease states.

Quite surprisingly we have discovered that another combination has demonstrated superior end-use characteristics than that of a synergistic effect in the treatment of malignant melanoma. This new aspect is the use of Interferon and Dacarbazine in conjunctive therapy with irreversible ODC inhibitors. This discovery is all the more surprising in view of the generally acknowledged disappointment of Interferon as an effective chemotherapeutic agent in diseases of this nature.

According to the concepts of this invention, the improvement in the treatment of rapidly proliferating tumor tissue disease states is generic in nature in that it relates to any ornithine decarboxylase inhibitor found to be useful in treating such disease states. However, in the present state of the art the particularly preferred ODC inhibitors with which Interferon and Dacarbazine can be conjunctively administered for the treatment of tumor tissue diseases are methyl acetylenic putrescine and compounds of the formulae

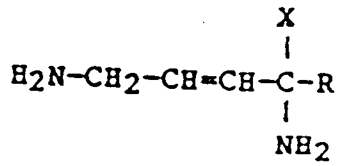 I

and

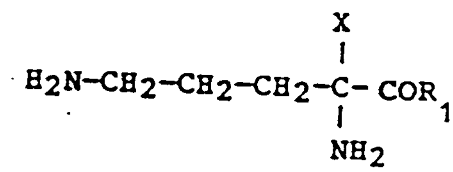

II

wherein

X is $-CHF_2$ or $-CH_2F$, and

R is H or $COR_1$, wherein

$R_1$ is OH or $C_{1-6}$ lower alkoxy,

and the pharmaceutically acceptable salts and individual optical isomers thereof. Particularly preferred compounds are α-difluoromethyl ornithine (α-DFMO), the methyl and ethyl esters of monofluoromethyl dehydroornithine, and the R,R-isomer of methyl acetylenic putrescine (i.e., (2R, 5R)-6-heptyne-2,5-diamine).

This invention is directed to a pharmaceutical product for use in an improved treatment of malignant melanoma by effecting polyamine depletion with ornithine decarboxylase inhibitors which comprises conjunctive administration of Interferon and Decarbazine.

According to the concepts of this invention the term "Interferon" is used in its generic meaning. Although known for over twenty years, Interferon has not yet been sufficiently characterized as to be subject to a precise definition and thus some difficulty arises in the use of the term. However, it is a term that is attached to those glycoproteins which are made by all animal cells after viral attack, and which function as regulating functionaries of the immune system. Interferon has also been made biosynthetically by recombinant DNA techniques and thus the precise characterization and/or nomenclature of such substance has been further complicated. However, as used in the art and in the context of this application, the term Interferon is meant to include all those natural and biosynthetic interferons capable of immunomodulation and include Type I and Type II interferons as well as the recombinant DNA types. Type I interferons, sometimes called leucocyte interferons include those α and β interferons generally prepared from leucocytes by viral infection. Type II interferons, sometimes called immunoactive interferon are those which are generally prepared from lymphocytic reactions with mitogens. Although, as stated above, the recombinant interferons are included herein, the immune and leucocyte interferons are preferred. Also included within the concept of the present invention is a composition for the treatment of tumor tissue with ODC inhibitors, Dacarbazine and Interferon when the Interferon is generated by the use of Interferon inducers (e.g., tilorone and its related analogs known as interferon inducers) which, in situ, induce the release of Interferon.

Dacarbazine, 5-(3,3-Dimethyl-1-triazenyl)-1H-imidazole-4-carboxamide, DTIC, has long been used in the treatment of metastatic malignant melanoma. While quite effective, DTIC is toxic and frequently causes unpleasant side effects such as vomiting and nausea as well as more serious toxic reactions such as hemopoietic depression, anemia, leukopenia, thrombocytopenia, and bone-marrow depression. The ability to augment or maintain the therapeutic efficacy of DTIC while reducing its untoward effects would be highly desirable.

The ability of compounds to irreversibly inhibit ornithine decarboxylase in vivo can be demonstrated as follows: An aqueous solution of the appropriate compound is given orally or parenterally to male mice or rats. The animals are sacrificed 1 to 48 hours after administration of the compound, and the ventral lobes of the prostate removed and homogenized. The activity of ornithine decarboxylase is measured as generally described by E.A. Pegg and H.G. Williams-Ashman, Biochem. J. 108, 553-539 (1968).

As used herein, the term "malignant melanomas," refers to those malignant tumors of melanocyte origin which are highly prone to metastasis. Malignant melanomas spread rapidly to adjacent tissue and particularly to nearby lymph nodes and eventually to the internal organs. Malignant melanomas include lentigo maligna melanoma, superficial spreading melanoma, and modular melanoma.

The term "controlling the growth," as used herein, means slowing, interrupting, arresting, or stopping the growth and metastases of a malignant melanoma in a warm blooded animal; it being understood that treatment (controlling the growth) in a warm blooded animal with ODC inhibitors, Interferon, and Dacarbazine does not generally provide a "cure" for the tumor in the sense that necessarily the tumor tissue is destroyed or totally eliminated.

The effect of an ODC inhibitor for the control of the growth rate of rapidly proliferating tumor tissue, such as a malignant melanoma, can be assessed in standard animal tumor models. For example, the anti-tumor effect of α-difluoromethyl-ornithine (DFMO) has been demonstrated in the following animal tumor models: (a) L1210 leukemia in mice, (b) EMT6 tumor in Balb/C mice, (c) 7,12-dimethylbenzanthracene-induced (DMBA-induced) mammary tumor in rats, and (d) Morris 7288C or 5123 hepatoma in Buffalo rats. In addition, the anti-tumor effect of DFMO in combination with various cytotoxic agents has been demonstrated as follows: (a) in combination with vindesine or adriamycin in L1210 leukemia in mice, in Morris 7288C hepatoma in Buffalo rats, and in EMT6 tumor in mice, (b) in combination with cytosine arabinoside in L1210 leukemia in mice, (c) in combination with methotrexate in L1210 leukemia in mice, (d) in combination with cyclophosphamide in EMT6 tumor in mice and in DMBA-induced tumor in mice, (e) in combination with BCNU in mouse glioma 26 brain tumor, and (f) in combination with MGBG in L1210 leukemia in mice, in Morris 7288C hepatoma in Buffalo rats, in P388 lymphocytic leukemia in mice, and in S-180 sarcoma in mice.

The product of the present invention is particularly advantageous in that both Interferon and the ODC inhibitors are essentially non-toxic. The term "combination therapy" contemplates the administration of an ODC inhibitor immediately prior to the beginning of therapy with DTIC and Interferon concomitantly with such therapy, or during the period of time immediately following cessation of such therapy. Preferably, the patient is treated with an ODC inhibitor for about 1 to 14 days, preferably 4 to 14 days, prior to the beginning of therapy with DTIC and Interferon and, thereafter, on a daily basis during the course of such therapy. Daily treatment with the ODC inhibitor can be continued for a period of, for example, 1 to 365 days after the last dose of DTIC is administered.

When such combination therapy results in remission of the tumor, and all tumor cells are not destroyed, regrowth of the tumor may be prevented or slowed indefinitely by continued treatment with an ODC inhibitor. Thus, Interferon and an ODC inhibitor can be administered to stop or slow the growth of the tumor during the periods when therapy using DTIC may be temporarily discontinued.

Another application of the enhancement of treatment with the combination of an ODC inhibitor, Interferon, and DTIC is the enhanced treatment of "secondary" viral infections in those subjects who have lost their natural immunity from other treatments.

Suitable dosages of a α-DFMO or other ODC inhibitors for use in combination therapy with DTIC and Interferon can be any amount effective in inhibiting polyamine biosynthesis sufficiently to control the tumor growth rate or to achieve a heightened response to DTIC administered in conjunction therewith.

As pharmacologically useful agents, the ODC inhibitors can be administered in various manners to the patient being treated to achieve the desired effect. The compounds can be administered either alone or in combination with one another, or they can be administered in the form of pharmaceutical compositions, which are well known in the art. The compounds may be administered orally or parenterally (for example, intravenously, intraperitoneally, or subcutaneously), including injection of the active ingredient directly into the tumor. The amount of compound administered will vary over a wide range and can be any effective amount. Depending upon the patient to be treated, the severity of the condition being treated, the mode of administration, and the particular compound employed, the effective amount of compound administered will vary from about 1 mg/kg to 2000 mg/kg of body weight of the patient per day and preferably will be about 10 mg/kg to 500 mg/kg of body weight of patient per day. For example, a typical unit dosage form may be a tablet containing from 100 to 500 mg of a compound which may be administered to the patient being treated 1 to 10 times daily to achieve the desired effect.

The dosage of DTIC and Interferon used in the combination therapy of this invention will be any effective amounts, for example, the amounts of these agents employed when used individually. The conjunctive use of ODC inhibitors will then bring about an augmented response of these agents.

As used herein the term patient is taken to mean warm blooded animals such as mammals, for example, dogs, rats, mice, cats, guinea pigs, horses, bovine cows, sheep, and humans.

The solid unit dosage forms can be of the conventional type. Thus, the solid form can be a capsule

which can be of the ordinary gelatin type containing a novel compound of this invention and a carrier, for example, lubricant and inert fillers, such as lactose, sucrose and corn starch. In another embodiment, the novel compounds are tableted with conventional tablet bases such as lactose, sucrose, or corn starch in combination with binders such as acacia, corn starch or gelatin, disintegrating agents such as corn starch, potato starch, or alginic acid, and a lubricant such as stearic acid, or magnesium stearate.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable or synthetic origin, for example, peanut oil, soybean oil and mineral oil. In general, water, saline, aqueous dextrose and related sugar solutions, ethanol and glycols such as propylene glycol or polyethylene glycol are preferred liquid carriers, particularly for injectable solutions.

The results of ODC inhibitors in the treatment of malignant melanomas when used in combination with Interferon (IFN) and DTIC have demonstrated a striking tumor suppression and such results can be shown and ascertained by standard and well-known laboratory techniques as may be illustrated by the following.

EXAMPLE I

EFFECT OF A COMBINATION OF α-DIFLUOROMETHYLORNITHINE (DFMO), DACARBAZINE (DTIC) AND α-INTERFERON (IFN) ON THE GROWTH OF B16 MELANOMA IN MICE

| Treatment | Tumor Weight (g) (Mean ± S.E.) | % Inhibition |
|---|---|---|
| Control | 7.23 ± 0.33 | |
| DFMO (2%) | 1.02 ± 0.15 | 86.0 |
| DTIC (6.25 mg/kg) | 3.2 ± 0.63 | 35.0 |
| IFN | 2.49 ± 0.53 | 65.0 |
| DFMO + DTIC | 0.60 ± 0.16 | 92.0 ** |
| IFN + DTIC | 0.55 ± 0 .61 | 92.0 |
| DFMO + IFN + DTIC | 0.10 ± 0.04* | 99.0** |

* 4 out of 6 animals had microscopic tumors weighing less than 50 mg.

** Statistically significant at $P<0.001$ compared to DFMO group.

B16 melanoma cells ($1 \times 10^5$ cells/animal) were injected S.C. in the interscapular region. DFMO was administered as 2% solution in the drinking water from day 1-day 18. DTIC was administered at a dose of 6.25 mg/Kg, i.p. once daily from day 1-day 15. IFN was administered S.C. at a dose of 1000 units/animal on alternate days from day 2-day 18. Combination treatments were administered as indicated. The animals were sacrificed on day 18, and the tumors were dissected and weighed.

EXAMPLE 2

Sterile Lyophillized Powder for Reconstitution

| | |
|---|---|
| α-DFMO | 2000 mg. |
| Interferon | $10^5$ to $10^6$ units |

To be reconstituted with 20 ml. water for injection just prior to infusion.

EXAMPLE 3

Preconstituted Sterile Solution for Infusion

| | |
|---|---|
| α-DFMO | 2000 mg. |
| Interferon | $10^5$ to $10^6$ units |
| Water for injection | q.s. 20 ml. |

The α-DFMO being a strong buffer agent in the acid range, enchances the stability of the interferon.

Of course, the ODC inhibitors DTIC and Interferon can be administered separately depending upon the judgement of the attending physician.

The term "conjunctive therapy" is intended to cover simultaneous or sequential administration of the active principles in such a way that the pharmacological effects of a substance are not completely exstinguished when the other is administered.

**Claims**

**1.** A pharmaceutical product containing an ornithine decarboxylase inhibitor, Interferon and Dacarbazine as a combined preparation for simultaneous or sequential use in treating rapidly-proliferating cell-growth disease states.

**2.** A product according to claim 1 for use as a cytostatic agent.

**3.** A product according to claim 1 for affecting polyamine depletion.

**4.** A product according to claim 1 for use in the treatment of malignant melanomas.

**5.** A product according to claim 1, 2, 3 or 4 wherein the ornithine decarboxylase inhibitor is alpha-DMFO or a pharmaceutically acceptable salt thereof.

**6.** A product according to claim 1, 2, 3 or 4 wherein the ornithine decarboxylase inhibitor is the methyl ester of mono-fluoromethyl dehydroornithine or a pharmaceutically acceptable salt thereof.

**7.** A product according to claim 1, 2, 3 or 4 wherein the ornithine decarboxylase inhibitor is the R,R-isomer of methyl acetylenic putrescine or a pharmaceutically acceptable salt thereof.

**8.** A pharmaceutical product containing an ornithine decarboxylase inhibitor and Dacarbazine as a combined preparation for simultaneous or sequential use in treating rapidly-proliferating cell-growth disease states.

**9.** A product according to claim 8 for use as a cytostatic agent.

**10.** A product according to claim 8 for affecting polyamine depletion.

**11.** A product according to claim 8 for use in the treatment of malignant melanomas.

**12.** A product according to claim 8, 9, 10 or 11 wherein the ornithine decarboxylase inhibitor is alpha-DMFO or a pharmaceutically acceptable salt thereof.

**13.** A product according to claim 8, 9, 10 or 11 wherein the ornithine decarboxylase inhibitor is the methyl ester of mono-fluoromethyl dehydroornithine or a pharmaceutically acceptable salt thereof.

**14.** A product according to claim 8, 9, 10 or 11 wherein the ornithine decarboxylase inhibitor is the R,R-isomer of methyl acetylenic putrescine or a pharmaceutically acceptable salt thereof.

**15.** Use of an ornithine decarboxylase inhibitor, Interferon and/or Dacarbazine in the manufacture of a combined pharmaceutical preparation for simultaneous or sequential use in treating rapidly-proliferating cell-growth disease states.

**16.** Use according to claim 15 wherein the preparation is for use as a cytostatic agent.

17. Use according to claim 15 wherein the preparation is for affecting the polyamine depletion.

18. Use according to claim 15 wherein the preparation is for use in the treatment of malignant melanomas.

19. Use according to claim 15, 16, 17 or 18 wherein the ornithine decarboxylase inhibitor is alpha-DMFO or a pharmaceutically acceptable salt thereof.

20. Use according to claim 15, 16, 17 or 18 wherein the ornithine decarboxylase inhibitor is the methyl ester of mono-fluoromethyl dehydroornithine or a pharmaceutically acceptable salt thereof.

21. Use according to claim 15, 16, 17 or 18 wherein the ornithine decarboxylase inhibitor is the R,R-isomer of methyl acetylenic putrescine or a pharmaceutically acceptable salt thereof.

22. A process for preparing a product according to claim 1, 2, 3, 4, 5, 6 or 7 which comprises preparing or formulating an ornithine decarboxylase inhibitor, Interferon and/or Dacarbazine for simultaneous or sequential administration.

**Revendications**

1. Produit pharmaceutique contenant un inhibiteur de l'ornithine-décarboxylase, de l'Interféron et de la Dacarbazine, en tant que préparation associée, pour usage simultané ou séquentiel dans le traitement des pathologies à croissance et prolifération cellulaires rapides.

2. Produit selon la revendication 1 pour usage comme agent cytostatique.

3. Produit selon la revendication 1 destiné à provoquer la déplétion des polyamines.

4. Produit selon la revendication 1, pour usage dans le traitement des mélanomes malins.

5. Produit selon la revendication 1, 2, 3 ou 4, dans lequel l'inhibiteur de l'ornithine-décarboxylase est de l'α-DMFO ou un sel pharmaceutiquement acceptable de cette dernière.

6. Produit selon la revendication 1, 2, 3 ou 4, dans lequel l'inhibiteur de l'ornithine-décarboxylase est l'ester méthylique de la monofluorométhyldéhydro-ornithine ou un sel pharmaceutiquement acceptable de ce dernier.

7. Produit selon la revendication 1, 2, 3 ou 4, dans lequel l'inhibiteur de l'ornithine-décarboxylase est l'isomère R,R de la putrescine méthylacétylénique ou un sel pharmaceutiquement acceptable de ce dernier.

8. Produit pharmaceutique contenant un inhibiteur de l'ornithine-décarboxylase et de la Dacarbazine, en tant que préparation associée, pour usage simultané ou séquentiel dans le traitement des pathologies à prolifération et croissance cellulaires rapides.

9. Produit selon la revendication 8 pour usage comme agent cytostatique.

10. Produit selon la revendication 8 destiné à provoquer la déplétion des polyamines.

11. Produit selon la revendication 8 pour usage dans le traitement des mélanomes malins.

12. Produit selon la revendication 8, 9, 10 ou 11, dans lequel l'inhibiteur de l'ornithine-décarboxylase est de l'α-DMFO OU un sel pharmaceutiquement acceptable de cette dernière.

13. Produit selon la revendication 8, 9, 10 ou 11, dans lequel l'inhibiteur de l'ornithine-décarboxylase est l'ester méthylique de la monofluorométhyldéhydro-ornithine ou un sel pharmaceutiquement acceptable de ce dernier.

14. Produit selon la revendication 8, 9, 10 ou 11, dans lequel l'inhibiteur de l'ornithine-décarboxylase est

l'isomère R,R de la putrescine méthylacétylénique ou un sel pharmaceutiquement acceptable de ce dernier.

15. Emploi d'un inhibiteur de l'ornithine-décarboxylase, de l'Interféron et/ou de la Dacarbazine dans la production d'une préparation pharmaceutique associée, pour usage simultané ou séquentiel dans le traitement des pathologies à prolifération et croissance cellulaires rapides.

16. Emploi selon la revendication 15, dans lequel la préparation est destinée à être utilisée comme agent cytostatique.

17. Emploi selon la revendication 15, dans lequel la préparation est destinée à provoquer la déplétion des polyamines.

18. Emploi selon la revendication 15, dans lequel la préparation est destinée à l'usage dans le traitement des mélanomes malins.

19. Emploi selon la revendication 15, 16, 17 ou 18, dans lequel l'inhibiteur de l'ornithine-décarboxylase est de l'$\alpha$-DMFO ou un sel pharmaceutiquement acceptable de cette dernière.

20. Emploi selon la revendication 15, 16, 17 ou 18, dans lequel l'inhibiteur de l'ornithine-décarboxylase est l'ester méthylique de la monofluométhyldéhydro-ornithine ou un sel pharmaceutiquement acceptable de ce dernier.

21. Emploi selon la revendication 15, 16, 17 ou 18, dans lequel l'inhibiteur de l'ornithine-décarboxylase est l'isomère R,R de la putrescine méthylacétylénique ou un sel pharmaceutiquement acceptable de ce dernier.

22. Procédé de préparation d'un produit selon la revendication 1, 2, 3, 4, 5, 6 ou 7, comprenant la préparation ou la formulation d'un inhibiteur de l'ornithine-décarboxylase, de l'Interféron et/ou de la Dacarbazine pour administration simultanée ou séquentielle.

**Patentansprüche**

1. Pharmazeutisches Produkt mit einem Gehalt an Ornithin-decarboxylase-Inhibitor, Interferon und Dacarbazin als kombiniertes Präparat zur gleichzeitigen oder aufeinanderfolgenden Verwendung bei der Behandlung von Krankheitszuständen mit rasch wucherndem Zellwachstum.

2. Produkt nach Anspruch 1 zur Verwendung als zytostatisches Mittel.

3. Produkt nach Anspruch 1 zur Beeinflussung der Polyamin-Erschöpfung.

4. Produkt nach Anspruch 1 zur Verwendung bei der Behandlung von malignen Melanomen.

5. Produkt nach Anspruch 1, 2, 3 oder 4, wobei es sich beim Ornithin-decarboxylase-Inhibitor um $\alpha$-DMFO oder ein pharmazeutisch verträgliches Salz davon handelt.

6. Produkt nach Anspruch 1, 2, 3 oder 4, wobei es sich beim Ornithin-decarboxylase-Inhibitor um den Methylester von Monofluormethyldehydroornithin oder ein pharmazeutisch verträgliches Salz davon handelt.

7. Produkt nach Anspruch 1, 2, 3 oder 4, wobei es sich beim Ornithin-decarboxylase-Inhibitor um das R,R-Isomere von Methylacetylenputrescin oder ein pharmazeutisch verträgliches Salz davon handelt.

8. Pharmazeutisches Produkt mit einem Gehalt an einem Ornithin-decarboxylase-Inhibitor und Dacarbazin als kombiniertes Präparat zur gleichzeitigen oder aufeinanderfolgenden Verwendung bei der Behandlung von Krankheitszuständen mit rasch wucherndem Zellwachstum.

9. Produkt nach Anspruch 8 zur Verwendung als zytostatisches Mittel.

**10.** Produkt nach Anspruch 8 zur Beeinflussung der Polyamin-Erschöpfung.

**11.** Produkt nach Anspruch 8 zur Verwendung bei der Behandlung von malignen Melanomen.

**12.** Produkt nach Anspruch 8, 9, 10 oder 11, wobei es sich beim Ornithin-decarboxylase-Inhibitor um α-DMFO oder ein pharmazeutisch verträgliches Salz davon handelt.

**13.** Produkt nach Anspruch 8, 9, 10 oder 11, wobei es sich beim Ornithin-decarboxylase-Inhibitor um den Methylester von Monofluormethyldehydroornithin oder ein pharmazeutisch verträgliches Salz davon handelt.

**14.** Produkt nach Anspruch 8, 9, 10 oder 11, wobei es sich beim Ornithin-decarboxylase-Inhibitor um das R,R-Isomere von Methylacetylenputrescin oder ein pharmazeutisch verträgliches Salz davon handelt.

**15.** Verwendung von einem Ornithin-decarboxylase-Inhibitor, Interferon und/oder Dacarbazin bei der Herstellung eines kombinierten pharmazeutischen Präparats zur gleichzeitigen oder aufeinanderfolgenden Anwendung bei der Behandlung von Krankheitszuständen mit rasch wucherndem Zellwachstum.

**16.** Produkt nach Anspruch 15 zur Verwendung als zytostatisches Mittel.

**17.** Produkt nach Anspruch 15 zur Beeinflussung der Polyamin-Erschöpfung.

**18.** Produkt nach Anspruch 15 zur Verwendung bei der Behandlung von malignen Melanomen.

**19.** Produkt nach Anspruch 15, 16, 17 oder 18, wobei es sich beim Ornithin-decarboxylase-Inhibitor um α-DMFO oder ein pharmazeutisch verträgliches Salz davon handelt.

**20.** Produkt nach Anspruch 15, 16, 17 oder 18, wobei es sich beim Ornithin-decarboxylase-Inhibitor um den Methylester von Monofluormethyldehydroornithin oder ein pharmazeutisch verträgliches Salz davon handelt.

**21.** Produkt nach Anspruch 15, 16, 17 oder 18, wobei es sich beim Ornithin-decarboxylase-Inhibitor um das R,R-Isomere von Methylacetylenputrescin oder ein pharmazeutisch verträgliches Salz davon handelt.

**22.** Verfahren zur Herstellung eines Produkts gemäß Anspruch 1, 2, 3, 4, 5, 6 oder 7, umfassend die Bereitstellung oder Formulierung eines Ornithin-decarboxylase-Inhibitor, von Interferon und/oder Dacarbazin zur gleichzeitigen oder aufeinanderfolgenden Verabreichung.